(19) Europäisches Patentamt
European Patent Office
Office européen des brevets

(11) **EP 1 567 465 B1**

(12) **EUROPEAN PATENT SPECIFICATION**

(45) Date of publication and mention
of the grant of the patent:
**30.03.2016 Bulletin 2016/13**

(51) Int Cl.:
*C07C 2/66* (2006.01)      *C07C 6/12* (2006.01)
*C07C 15/02* (2006.01)     *C07C 15/073* (2006.01)
*C07C 15/085* (2006.01)

(21) Application number: **03747580.3**

(22) Date of filing: **08.04.2003**

(86) International application number:
**PCT/US2003/010724**

(87) International publication number:
**WO 2003/093230 (13.11.2003 Gazette 2003/46)**

(54) **PROCESS FOR AROMATICS ALKYLATION EMPLOYING ZEOLITE BETA PREPARED BY THE IN-EXTRUDATE METHOD**

VERFAHREN ZUR ALKYLIERUNG AROMATISCHER VERBINDUNGEN UNTER VERWENDUNG VON DURCH DAS IM-EXTRUDAT-VERFAHREN HERGESTELLTEM ZEOLIT-BETA

PROCÉDÉ D'ALKYLATION D'HYDROCARBURES AROMATIQUES AU MOYEN DE ZEOLITE BETA PRÉPARÉ PAR LE PROCÉDÉ DANS L'EXTRUDAT

(84) Designated Contracting States:
**BE DE FR IT NL**

(30) Priority: **30.04.2002 US 138061**

(43) Date of publication of application:
**31.08.2005 Bulletin 2005/35**

(73) Proprietor: **Chevron U.S.A. Inc.
San Ramon, CA 94583 (US)**

(72) Inventor: **MILLER, Stephen, J.
San Francisco, CA 94121 (US)**

(74) Representative: **Nash, David Allan et al
Haseltine Lake LLP
Redcliff Quay
120 Redcliff Street
Bristol BS1 6HU (GB)**

(56) References cited:
**US-A- 4 891 458    US-A- 5 558 851**

• **A. I. Biaglow ET AL: "A Study of Dealuminated Faujasites", Journal of Catalysis, vol. 148, no. 1, 1 July 1994 (1994-07-01), pages 213-223, XP055194826, DOI: 10.1006/jcat.1994.1203**

Remarks:
The file contains technical information submitted after the application was filed and not included in this specification

EP 1 567 465 B1

**Description**

FIELD OF THE INVENTION

This invention relates to a process for alkylation of aromatics employing zeolite beta prepared by the in-extrudate method, and more particularly to a process for the synthesis of cumene employing this alkylation method.

BACKGROUND OF THE INVENTION

Aromatic alkylation, particularly to produce cumene and ethylbenzene, may occur using a variety of different methods. At one time, benzene was alkylated with $C_2$ to $C_4$ olefins using the Friedel-Crafts method. Suitable Friedel-Crafts catalysts include aluminum chloride, boron trifluoride, hydrofluoric acid, liquid and solid phosphoric acid, sulfuric acid, etc. These materials are highly corrosive to process equipment, create operational problems, and are often difficult to dispose of in an environmentally acceptable manner.

Non-corrosive, solid catalysts, such as zeolites, have been used for aromatic alkylation as well. Catalysts which comprise zeolite beta, ZSM-5, X or Y zeolites have all been used in cumene manufacture. There have been difficulties involving cumene selectivity, catalyst life and ease of regeneration with many of these zeolites, however.

Zeolite beta, disclosed in U.S. Pat. No. 3,308,069, is a porous crystalline synthetic material having the following composition:

$$[(x/n)M(1+0.1-x)TEA]AlO_2 \cdot ySiO_2 \cdot wH_2O]$$

wherein x is smaller than 1, y is comprised within the range of from 5 to 100, w is comprised within the range of from 0 to 4, M is a metal belonging to the Groups IA, IIA, IIIA or is a transition metal, and TEA is tetraethyl-ammonium.

U.S. Pat. No. 4,891,458 discloses a process for the alkylation of an aromatic hydrocarbon by contacting a stoichiometric excess of the aromatic hydrocarbon with a $C_2$ to $C_4$ olefin under at least partial liquid phase conditions and in the presence of a catalyst comprising zeolite beta. U.S. Pat. No. 5,081,323 is a continuation of U.S. Pat. 4,891,458, and discloses the reaction occurring in two catalyst beds or reactors in series, with at least a portion of the aromatic hydrocarbon being added between the catalyst beds or reactors.

SUMMARY OF THE INVENTION

The present invention relates to an aromatic alkylation process which employs a catalyst comprising a new particulate beta zeolite which is useful for acid catalyzed hydrocarbon conversion reactions. The catalyst possesses properties of: a $SiO_2/Al_2O_3$ mole ratio of greater than 15; a water adsorption capacity of greater than 12 wt. %; a microporosity of greater than 0.13 cc/gm; and a total acidity greater than 0.7 mmole/gm of catalyst. This catalyst is prepared by the in-extrudate method, which is disclosed in U.S. Pat. No. 5,558,851.

This catalyst has excellent regeneration capacity for the production of cumene or ethylbenzene from the alkylation of benzene with propylene or ethylene. It also possesses higher activity and longer catalyst life than zeolite beta catalyst prepared by previous techniques. Furthermore, it has a high selectivity for cumene.

The alkylation process is described generally below:

A process for the alkylation of an aromatic hydrocarbon to produce at least one product, the process comprising contacting a stoichiometric excess of the aromatic hydrocarbon with a $C_2$ to $C_4$ olefin under at least partial liquid phase conditions with a catalyst comprising zeolite beta which has been prepared by a process comprising the following steps:

(a) preparing a reaction mixture comprising at least one active source of silica, optionally at least one active source of silica, optionally at least one active source of alumina, an organic templating agent capable of forming said crystalline zeolite, and sufficient water to shape said mixture; and

(b) heating said reaction mixture at crystallization conditions and in the absence of an external liquid phase for sufficient time to form a crystallized material containing crystals of said zeolite, wherein said zeolite crystals have a silica/alumina molar ratio greater than 15.

DETAILED DESCRIPTION OF THE INVENTION

Catalyst

Zeolite beta is a known synthetic crystalline aluminosilicate originally described in U.S. Pat. Nos. 3,308,069 and Re 28,341, to which reference is made for further details of this zeolite, its preparation and properties. Zeolite beta is a large pore zeolite having a constraint index of less than 1. In the instant invention, zeolite beta is prepared using the "in-extrudate" method, which is described in U.S. Pat. No. 5,558,851. The catalysts of the instant invention possess a higher water absorptive capacity and acidity.

The catalyst comprising zeolite beta is prepared from a reaction mixture having the following composition ranges:

$YO_2/W_2O_3$ = >15; preferably 15-30
$M+/YO_2$ = 0.03-0.5, preferably 0.05-0.3
$R/YO_2$ = 0.07-0.30, preferably 0.10-0.20
$OH-/YO_2$ = 0.10-0.30, preferably 0.12-0.25
$H_2O/YO_2$ = 1.5-4.0, preferably 1.7-3.2

Where Y is silicon; W is aluminum; M+ is an alkali metal ion; and R is a templating agent.

Zeolites may be crystallized within the reaction mixture or within the shaped particles made from the reaction mixture. Crystallization of the zeolite takes place in the absence of an external liquid phase, i.e., in the absence of a liquid phase separate from the reaction mixture. A more detailed description of the method of preparation of in-extrudate zeolites, including crystallization steps, is found in U.S. Pat. No. 5,558,851.

Suitable templating agents are organic cations which are derived in aqueous solution from tetraethylammonium bromide or hydroxide, dibenzyl-1,4-diazabicyclo[2.2.2]octane chloride, dimethyldibenzyl ammonium chloride, 1,4-di(1-azonium bicyclo[2.2.2]octane)butane dibromide or dihydroxide, and the like. These organic cations are known in the art and are described, for example, in European Patent Applications Nos. 159,846 and 159,847, and U.S. Pat. No. 4,508,837. The preferred organic cation is the tetraethylammonium ion. Typically, the templating agent will be an organic compound which contains nitrogen or phosphorus. The sources of organic nitrogen-containing cations may be primary, secondary, or tertiary amines or quaternary ammonium amines. Templating agents are discussed in more detail in U.S. Pat. No. 5,558,851.

M is typically a sodium ion from the original synthesis but may also be a metal ion added by ion exchange techniques. Suitable metal ions include those from Groups IA, IIA or IIIA of the Periodic Table or a transition metal. Examples of such ions include ions of lithium, potassium, calcium, magnesium, barium, lanthanum, cerium, nickel, platinum, palladium, and the like.

For high catalytic activity, the zeolite beta should be predominantly in its hydrogen ion form. Generally, the zeolite is converted to its hydrogen form by ammonium exchange followed by calcination. If the zeolite is synthesized with a high enough ratio of organonitrogen cation to sodium ion, calcination alone may be sufficient. It is preferred that, after calcination, hydrogen ions and/or rare earth ions occupy a major portion of the cation sites. It is especially preferred that at least hydrogen ions and/or rare earth ions occupy 80% of the cation sites.

The zeolite, as synthesized in shaped form, may be used as a catalyst. The shaped form within which the zeolite is crystallized may contain inorganic oxide binders, by adding one or more of those binders to the zeolite reaction mixture. It is believed that the addition of these binder materials improves intra-particle diffusion. The final catalyst may contain from 70 to 100 wt. % zeolite beta. Usually, the zeolite beta content will range from 80 to 100 wt. %, and more typically from 90 to 100 wt. %. The preferred inorganic binder is alumina. The mixture, prior to crystallization, may be formed into tablets or extrudates having the desired shape by methods well known in the art. The extrudates or tablets will usually be cylindrical in shape. With cross-sectional diameters ranging from 1/64 to 1/2 inch (0.40 to 12.7 mm), other shapes with enhanced surface-to-volume ratios, such as fluted or polylobed cylinders, can be employed to enhance mass transfer rates and, thus, catalytic activity.

Feeds

Examples of suitable aromatic hydrocarbon feedstocks which may be alkylated or transalkylated by the process of the invention include aromatic compounds such as benzene, toluene and xylene. The preferred aromatic hydrocarbon is benzene. Mixtures of aromatic hydrocarbons may also be employed.

Suitable olefins for the alkylation of the aromatic hydrocarbon are those containing 2 to 4 carbon atoms, such as ethylene, propylene, butene-1, trans-butene-2 and cis-butene-2, or mixtures thereof. Preferred olefins are ethylene and propylene. An especially preferred olefin is propylene. These olefins may be present in admixture with the corresponding $C_2$ to $C_4$ paraffins, but it is usually preferable to remove dienes, acetylenes, water, sulfur compounds or nitrogen compounds which may be present in the olefin feedstock stream, to prevent rapid catalyst deactivation. In some cases, however, it may be desirable to add, in a controlled fashion, small amounts of water or nitrogen compounds to optimize catalytic properties.

When transalkylation is desired, the transalkylating agent is a polyalkyl aromatic hydrocarbon containing two or more alkyl groups that each may have from 2 to about 4 carbon atoms. For example, suitable polyalkyl aromatic hydrocarbons include di-, tri- and tetra-alkyl aromatic hydrocarbons, such as diethylbenzene, triethylbenzene, diethylmethylbenzene (diethyltoluene), diisopropylbenzene, triisopropylbenzene, diisopropyltoluene, dibutylbenzene, and the like. Preferred polyalkyl aromatic hydrocarbons are the dialkyl benzenes. A particularly preferred polyalkyl aromatic hydrocarbon is diisopropylbenzene.

Reaction products which may be obtained from the process of the invention include ethylbenzene from the reaction of benzene with either ethylene or polyethylbenzenes, cumene from the reaction of benzene with propylene or polyisopropylbenzenes, ethyltoluene from the reaction of toluene with ethylene or polyethyltoluenes, cymenes from the reaction of toluene with propylene or polyisopropyltoluenes, and sec-butylbenzene from the reaction of benzene and n-butenes or polybutylbenzenes. The production of cumene from the alkylation of benzene with propylene or the transalkylation of benzene with di-isopropylbenzene is especially preferred.

Conditions

When alkylation is the process conducted according to this invention, reaction conditions are as follows. The aromatic hydrocarbon feed should be present in stoichiometric excess. It is preferred that the molar ratio of aromatics to olefins be at least about four to one (4:1) to prevent rapid catalyst fouling. The reaction temperature may range from 100°F to 600°F (37.8 to 315.6°C), preferably 250°F to 450°F (121.1 to 232.2°C). In the case of cumene production, a temperature range of 250°F to 375°F (121.1 to 190.6°C) is most preferred to reduce product impurities. The reaction pressure should be sufficient to maintain at least a partial liquid phase in order to retard catalyst fouling. This is typically a gauge pressure of 50 to 1000 psi (345 to 6900 kPa) depending on the feedstock and reaction temperature. Contact time may range from 10 seconds to 10 hours, but is usually from 5 minutes to an hour. The weight hourly space velocity (WHSV), in terms of grams (pounds) of aromatic hydrocarbon and olefin per gram (pound) of catalyst per hour, is generally within the range of about 0.5 to 50.

When transalkylation is the process conducted according to the invention, the molar ratio of aromatic hydrocarbon to polyalkyl aromatic hydrocarbon will generally range from about 1:1 to about 50:1, and preferably from about 2:1 to about 20:1. The reaction temperature may range from about 100°F to 600°F (37.8 to 315.6°C), but it is preferably about 250°F to 450°F (121.1 to 232.2°C). The reaction pressure should be sufficient to maintain at least a partial liquid phase, typically a gauge pressure in the range of about 50 psi to 1000 psi (345 to 6900 kPa), preferably 300 psig to 600 psig (2070 to 4140 kPa). The weight hour space velocity will range from about 0.1 to 10.

Various types of reactors can be used in the process of this invention. For example, the process can be carried out in batchwise fashion by adding the catalyst and aromatic feedstock to a stirred autoclave, heating to reaction temperature, and then slowly adding the olefinic or polyalkylaromatic feedstock. A heat transfer fluid can be circulated through the jacket of the autoclave, or a condenser can be provided, to remove the heat of reaction and maintain a constant temperature. Large scale industrial processes may employ a fixed bed reactor operating in an upflow or downflow mode or a moving bed reactor operating with concurrent or countercurrent catalyst and hydrocarbon flows. These reactors may contain a single catalyst bed or multiple beds and may be equipped for the interstage addition of olefins and interstage cooling. Interstage olefin addition and more nearly isothermal operation enhance product quality and catalyst life. A moving bed reactor makes possible the continuous removal of spent catalyst for regeneration and replacement by fresh or regenerated catalysts.

In a preferred embodiment of the present invention, the alkylation process is carried out with addition of olefin in at least two stages. Preferably, there will be two or more catalyst beds or reactors in series, wherein at least a portion of the olefin is added between the catalyst beds or reactors. Interstage cooling can be accomplished by the use of a cooling coil or heat exchanger. Alternatively, interstage cooling can be affected by staged addition of the aromatic feedstock, that is, by addition of the aromatic feedstock in at least two stages. In this instance, at least a portion of the aromatic feedstock is added between the catalyst beds or reactors, in similar fashion to the staged addition of olefin described above. The staged addition of aromatic feedstock provides additional cooling to compensate for the heat of reaction.

In a fixed bed reactor or moving bed reactor, alkylation is completed in a relatively short reaction zone following the introduction of olefin. Ten to thirty percent of the reacting aromatic molecules may be alkylated more than once.

The alkylation reactor effluent contains the excess aromatic feed, monoalkylated product, polyalkylated products, and various impurities. The aromatic feed is recovered by distillation and recycled to the alkylation reactor. Usually, a small bleed is taken from the recycle stream to eliminate unreactive impurities from the loop. The bottoms from the benzene distillation are further distilled to separate monoalkylated product from polyalkylated products and other heavies.

In most cases, the recovered monoalkylated product must be very pure. For example, current specifications call for 99.9% cumene purity with less than 500 ppm each of ethylbenzene and butylbenzene. Since only a small fraction of by-product ethylbenzene and n-propylbenzene can be economically removed by distillation, it is important to have a feedstock containing very little ethylene and a catalyst which makes very small amounts of these impurities.

The following examples are provided to illustrate the invention in accordance with the principles of the invention but are not to be construed as limiting the invention in any way except as indicated by the appended claims.

EXAMPLES

Example 1

To 120 grams of silica (Hi-Sil 233, a hydrated silica manufactured by PPG) were added 18 grams of $NaAlO_2$ in a Baker-Perkins mixer and mixed for 10 minutes. To this was added 150 grams of a 40 wt. % aqueous solution of tetraethylammonium hydroxide (TEAOH) and 8 grams of a 50 wt. % aqueous solution of NaOH. This mixture was mixed for three hours. Then 25 grams of water was added with mixing to bring the mixture to a paste. Another 30 grams of silica was added along with 7 grams of kaolin clay. Steam heat was applied to the jacket around the walls of the mixer to bring the mixture to an extrudable consistency at a volatiles level of 44% (by LOI). Molar ratios in the synthesis mix were as follows:

$$TEAOH+/SiO_2 = 0.16$$

$$OH-/SiO_2 = 0.20$$

$$Na+/SiO_2 = 0.14$$

$$SiO_2/Al_2O_3 = 17$$

$$H_2O/SiO_2 = 1.9$$

The mix was then extruded through a 1/16-inch (1.58 mm) die. The extrudate was placed in a Teflon bottle in a stainless steel pressure vessel and heated to 150°C at autogenous pressure for four days. The extrudate was washed with a 10% aqueous solution of ammonium nitrate containing 6 cc $HNO_3$ per 1000 grams of solution, dried overnight in a vacuum oven at 110°C, and calcined in air at 538°C for six hours. X-ray diffraction analysis showed the extrudate to be 96% beta. The micropore volume (by $N_2$ adsorption) was 0.20 cc/g and the surface area (by BET) was 568 $m^2$/g.

Example 2

The catalyst of Example 1 was tested in a down-flow micro-unit for cumene production. The catalyst was crushed to 24-42 mesh (0.465 to 0.85 mm sieve size) and calcined in air at 650°F (343.3°C) before installing the reactor in the micro-unit. The reactor was then pressured up to 600 psig (4140 kPa) using nitrogen and then lined out at a temperature of 300°F (148.9°C) prior to introducing feed. The feed was an 8/1 volumetric mixture of benzene/propylene, run at a WHSV of 5.7. A 4A mole sieve drier was installed in the feed line to remove any residual water. Samples were taken every three hours using an on-line gas chromatograph. Results at 40 hours on-stream are shown in Table I, along with results at the same conditions for a commercial prototype Beta catalyst made using conventional synthesis and containing 80% Beta zeolite bound with alumina. These results show the catalyst of Example 1 to give close to 95% selectivity to cumene at 100% propylene conversion.

| Table I Cumene Synthesis Test 8/1 Benzene/Propylene, 5.7 WHSV, 300°F (148.9°C), 600 psig (4140 kPa) (40 Hr On-stream) | | |
|---|---|---|
| Catalyst | Commercial | Example 1 |
| $SiO_2/Al_2O_3$ | 25 | 17 |
| Propylene Conv, % | 100 | 100 |
| Cumene, wt. % | 91.0 | 94.9 |
| 1,3 DIPB | 4.9 | 2.3 |

(continued)

| Table I |
| Cumene Synthesis Test 8/1 Benzene/Propylene, 5.7 WHSV, 300°F (148.9°C), 600 psig (4140 kPa) (40 Hr On-stream) |

| Catalyst | Commercial | Example 1 |
| --- | --- | --- |
| 1,4 DIPB | 3.7 | 2.1 |
| Other | 0.4 | 0.7 |

Example 3

To 1800 grams of silica (Hi-Sil 233, a hydrated silica manufactured by PPG) were added 78 grams of $NaAlO_2$ in a Baker-Perkins mixer. To this was added 1344 grams of a 35 wt. % aqueous solution of tetraethylammonium hydroxide (TEAOH), 144 grams of a 50 wt. % aqueous solution of NaOH, 114 grams alumina (Versal 250, manufactured by UOP, 74% $Al_2O_3$), 84 grams of kaolin clay, 145 grams of Beta zeolite powder as seed, then 1035 grams of water. This mixture was mixed for 20 minutes. Steam heat (160°F (71.1 °C)) was applied to the jacket around the walls of the mixer to bring the mixture to an extrudable consistency at a volatiles level of 53% (by LOI). The mix was then extruded through a 1/16-inch (1.58 mm) die, and air dried on trays to 43% LOI. Molar ratios in the synthesis mix were as follows:

$$TEAOH+/SiO_2 = 0.12$$

$$OH-/SiO_2 = 0.18$$

$$Na+/SiO_2 = 0.10$$

$$SiO_2/Al_2O_3 = 17$$

$$H_2O/SiO_2 = 2.3$$

The extrudate was placed in a jacketed rotating stainless steel pressure vessel and heated for 36 hours at autogenous pressure to 150°C by circulating hot oil through the jacket. The extrudate was washed with a 10% aqueous solution of ammonium nitrate containing 6 cc $HNO_3$ per 1000 grams of solution, dried overnight in a vacuum oven at 110°C, and calcined in air at 538°C for six hours. X-ray diffraction analysis showed the extrudate to be 97% beta.

Example 4

The catalyst of Example 3 was tested for cumene production using the same method as given in Example 2, except the WHSV was 41.6 to accelerate aging in order to determine catalyst cycle life. The run was considered over when the propylene conversion dropped below 95%. At 40 hours on-stream, cumene selectivity was 92%, and was 94% at 122 hours. The cycle life with this catalyst was 131 hours.

Comparative Example

The run of Example 4 was repeated, this time using a commercial Beta zeolite extrudate containing 80 wt. % Beta zeolite and 20% wt. % alumina binder. The cycle life with this catalyst was 43 hours.

Example 5

The catalyst of Example 4 was mildly crushed and pre-hydrated for seven days, then analyzed by NMR and found to have a water-free tetrahedral Al content of 3.1 wt. %, corresponding to a framework $SiO_2/Al_2O_3$ molar ratio of 27. This catalyst was then compared against a commercial Beta zeolite of the same framework $SiO_2/Al_2O_3$ molar ratio for water adsorption using the H Corr-Purcell-Meiboom-Gill (CPMG) method. This method is described in T.C Farrar and E.D. Becker, "Pulse and Fourier Transform NMR," 1971 Edition, Academic Press, New York. Results are shown in Table II.

**Table II**
**Percent Water Adsorption by H CPMG Method**

|  | $H_2O$, Wt. % | $H_2O$, Wt. % (repeat analysis) |
|---|---|---|
| Example 3 catalyst | 19.6 | 20.1 |
| Commercial Beta zeolite | 15.2 | 17.0 |

These results show the catalyst of Example 3 has greater capacity for water. The commercial beta zeolite in this example was not bound with alumina. Had the catalyst of Example 3 been compared against a conventional Beta catalyst, in which the zeolite is bound with alumina, the difference in $H_2O$ capacity would have been expected to be even greater.

Example 6

The acidity measurement by temperature programmed desorption of isopropylamine is applicable to determining the amount of Bronsted acid sites due to their strong interaction.

The chemically adsorbed isopropylamine molecules decompose to propylene at temperatures above 280°C. The amount of acid sites on a zeolite can be determined by using thermogravimetric technique (TGA) to measure the amount of propylene decomposed. In a thermogravimetric experiment, the sample is activated at a temperature of 500°C. Isopropylamine is adsorbed onto the zeolite. The sample is allowed to release any physisorbed isopropylamine, then heated to release the remaining chemisorbed isopropylamine.

Since each IPA molecule stoichiometrically interacts with one proton on the zeolite, the acid density or acidity can be calculated based on the amount of isopropylamine desorbed between 280°C and 500°C. The following procedure is carried out:

1. Load 25-35 milligrams of sample onto (tarred) platinum pan.

2. Insert balance arm into furnace portion of TGA.

3. Run TGA program (under constant He purge):

- Ramp to 500°C (@10°C/min.) and hold for 15 minutes to activate sample.
- Cool to 100°C (freefall) to allow adsorption of IPAm(isopropylamine).
- Introduce IPAm vapor until weight gain reaches a plateau, or for 15 minutes (adsorption of IPAm).
- Ramp to 280°C (@10°C/min.), hold for 30 minutes (to allow for desorption of physisorbed IPAm).
- Ramp to 500°C to allow for desorption of chemisorbed IPAm.

The residual weight of the sample is measured at 100°C, before introduction of IPAm (anhydrous weight) and at point right before ramp to 500°C (after desorption of physisorbed IPAm).

The total acid site density is obtained in mmol/g as follows:

$$\text{Acid site density (mmol/g)} = \frac{\text{Wt. of zeolite with chemisorbed IPAm} - \text{Anhydrous wt. of Zeolite}}{\text{Anhydrous wt. of Zeolite} \times \text{MW (IPAm)}}$$

The acidity of the catalyst of Example 3, as determined by the isopropylamine TPD test, was 0.97 meq/g. The same analysis done on a commercial Beta catalyst similar to that of Example 5 showed an acidity of 0.5 mmole/g.

**Claims**

1. A process for the alkylation of an aromatic hydrocarbon to produce at least one product, the process comprising contacting a stoichiometric excess of the aromatic hydrocarbon with a $C_2$ to $C_4$ olefin under at least partial liquid phase conditions with a catalyst comprising zeolite beta which has been prepared by a process comprising the following steps:

(a) preparing a reaction mixture comprising at least one active source of silica, optionally at least one active source of alumina, an organic templating agent capable of forming said crystalline zeolite, and sufficient water to shape said mixture; and

(b) heating said reaction mixture at crystallization conditions and in the absence of an external liquid phase for sufficient time to form a crystallized material containing crystals of said zeolite, wherein said zeolite crystals have a silica/alumina ratio greater than 15;

wherein the said catalyst has a water adsorption capacity of greater than 12 wt%, a microporosity of greater than 0.13 cc/gm and a total acidity greater than 0.7 mmole/gm;

wherein the total acidity is measured by temperature programmed desorption of isopropylamine between 280°C and 500°C carried out by the procedure set forth in Example 6 in the description; and

wherein the water adsorption capacity is measured using the H Corr-Purcell-Meiboom-Gill method.

2. The process of claim 1, wherein the molar ratio of aromatic hydrocarbon to olefin is at least 4:1.

3. The process of claim 1, wherein the aromatic hydrocarbon is selected from the group consisting of benzene, toluene, and xylene, or mixtures thereof.

4. The process of claim 1, wherein the aromatic hydrocarbon is benzene.

5. The process of claim 1, wherein the olefin is a member selected from the group consisting of ethylene, propylene, butene-1, trans-butene-2, and cis-butene-2 or mixtures thereof.

6. The process of claim 1, wherein the product of the alkylation is cumene or ethylbenzene.

7. A process for the transalkylation of an aromatic hydrocarbon, wherein a stoichiometric excess of an aromatic hydrocarbon is contacted with a polyalkyl aromatic hydrocarbon, under at least partial liquid phase conditions with a catalyst comprising zeolite beta which has been prepared by a process comprising the following steps:

(a) preparing a reaction mixture comprising at least one active source of silica, optionally at least one active source of alumina, an organic templating agent capable of forming said crystalline zeolite, and sufficient water to shape said mixture; and

(b) heating said reaction mixture at crystallization conditions and in the absence of an external liquid phase for sufficient time to form a crystallized material containing crystals of said zeolite, wherein said zeolite crystals have a silica/alumina molar ratio greater than 15;

wherein the said catalyst has a water adsorption capacity of greater than 12 wt%, a microporosity of greater than 0.13 cc/gm and a total acidity greater than 0.7 mmole/gm;

wherein the total acidity is measured by temperature programmed desorption of isopropylamine between 280°C and 500°C carried out by the procedure set forth in Example 6 in the description; and

wherein the water adsorption capacity is measured using the H Corr-Purcell-Meiboom-Gill method.

**Patentansprüche**

1. Alkylierungsverfahren für einen aromatischen Kohlenwasserstoff zum Herstellen mindestens eines Produkts, das Verfahren umfassend Zusammenbringen eines stoechiometrischen Überschusses des aromatischen Kohlenwasserstoffs mit einem $C_2$- bis $C_4$-Olefin unter mindestens teilweise Flüssigphasenbedingungen mit einem Katalysator, umfassend Zeolit-Beta, das hergestellt wurde mit einem Verfahren, umfassend die folgenden Schritte

(a) Herstellen eines Reaktionsgemischs, umfassend mindestens eine aktive Siliciumdioxidquelle, wahlweise mindestens eine aktive Aluminiumoxidquelle, ein organisches Templat, fähig zum Bilden des kristallinen Zeolits, und ausreichend Wasser zum Formen des Gemischs; und

(b) Erhitzen des Reaktionsgemischs bei Kristallisierungsbedingungen und in der Abwesenheit einer externen flüssigen Phase während ausreichend Zeit zum Bilden eines kristallisierten Materials, enthaltend Kristalle des Zeolits, wobei die Zeolitkristalle ein Siliciumdioxid-Aluminiumoxid-Verhältnis größer als 15 haben; wobei der Katalysator eines Wasseradsorptionskapazität größer als 12 Gew.-% hat, eine Mikroporosität größer

als 13 cm$^3$/g und eine Gesamtsäure größer als 0,7 mmol/g;

wobei die Gesamtsäure gemessen wird durch temperaturprogrammierte Desorption von Isopropylamin zwischen 280°C und 500°C, ausgeführt durch das Verfahren, wie in Beispiel 6 der Beschreibung beschrieben; und

wobei die Wasseradsorptionskapazität mit dem H Corr-Purcell-Meiboom-Gill-Verfahren gemessen wird.

**2.** Verfahren gemäß Anspruch 1, wobei das Molarverhältnis von aromatischem Kohlenwasserstoff zu Olefin mindestens 4:1 ist.

**3.** Verfahren gemäß Anspruch 1, wobei der aromatische Kohlenwasserstoff ausgewählt ist aus der Gruppe Benzol, Toluol und Xylol, oder deren Gemische.

**4.** Verfahren gemäß Anspruch 1, wobei der aromatische Kohlenwasserstoff Benzol ist.

**5.** Verfahren gemäß Anspruch 1, wobei das Olefin ein Element ist, ausgewählt aus der Gruppe Ethylen, Propylen, 1-Buten, trans-2-Buten und cis-2-Buten oder deren Gemische.

**6.** Verfahren gemäß Anspruch 1, wobei das Produkt der Alkylierung Cumol oder Ethylbenzol ist.

**7.** Verfahren für die Transalkylierung eines aromatischen Kohlenwasserstoffs, wobei ein stoechiometrischer Überschuss eines aromatischen Kohlenwasserstoffs mit einem Polyalkyl-aromatischen Kohlenwasserstoff zusammengebracht wird, unter mindestens teilweise Flüssigphasenbedingungen mit einem Katalysator, umfassend Zeolit-Beta, das hergestellt wurde mit einem Verfahren, umfassend die folgenden Schritte

(a) Herstellen eines Reaktionsgemischs, umfassend mindestens eine aktive Siliciumdioxidquelle, wahlweise mindestens eine aktive Aluminiumoxidquelle, ein organisches Templat, fähig zum Bilden des kristallinen Zeolits, und ausreichend Wasser zum Formen des Gemischs; und

(b) Erhitzen des Reaktionsgemischs bei Kristallisierungsbedingungen und in der Abwesenheit einer externen flüssigen Phase während ausreichend Zeit zum Bilden eines kristallisierten Materials, enthaltend Kristalle des Zeolits, wobei die Zeolitkristalle ein Siliciumdioxid-Aluminiumoxid-Verhältnis größer als 15 haben;

wobei der Katalysator eines Wasseradsorptionskapazität größer als 12 Gew.-% hat, eine Mikroporosität größer als 13 cm$^3$/g und eine Gesamtsäure größer als 0,7 mmol/g;

wobei die Gesamtsäure gemessen wird durch temperaturprogrammierte Desorption von Isopropylamin zwischen 280°C und 500°C, ausgeführt durch das Verfahren, wie in Beispiel 6 der Beschreibung beschrieben; und

wobei die Wasseradsorptionskapazität mit dem H Corr-Purcell-Meiboom-Gill-Verfahren gemessen wird.

## Revendications

**1.** Procédé pour l'alkylation d'un hydrocarbure aromatique pour produire au moins un produit, le procédé comprenant contacter un excès stoechiométrique de l'hydrocarbure aromatique avec une oléfine $C_2$ à $C_4$ dans des conditions au moins partiellement en phase liquide avec un catalyseur comprenant une zéolite-bêta qui a été préparée par un procédé comprenant les étapes suivantes :

(a) préparer un mélange réactionnel comprenant au moins une source active de silice, éventuellement au moins une source active d'alumine, un agent structurant organique capable de former ladite zéolite cristalline, et assez d'eau pour former ledit mélange ; et

(b) chauffer ledit mélange réactionnel dans des conditions de récristallisation et en l'absence d'une phase liquide externe pendant un temps suffisant pour former un matériau cristallin contenant des cristaux de ladite zéolite, où lesdits cristaux de zéolite ont un rapport silice/alumine supérieur à 15 ;

où ledit catalyseur a une capacité d'adsorption d'eau supérieure à 12 pour cent en poids, une microporosité supérieure à 13 cm$^3$/g et une acidité totale supérieure à 0,7 mmol/g ;

où l'acidité totale est mesurée par désorption programmée de température d'isopropylamine entre 280° et 500°C, exécutée par la procédure décrite dans l'Exemple 6 dans la description ; et

où la capacité d'adsorption d'eau est mesurée en utilisant le procédé de H Corr-Purcell-Meiboom-Gill.

**2.** Procédé selon la revendication 1, où le rapport molaire d'hydrocarbure aromatique et d'oléfine est au moins 4:1.

**3.** Procédé selon la revendication 1, où l'hydrocarbure aromatique est sélectionné parmi le groupe constitué en le benzène, le toluène, et le xylène, ou leurs mélanges.

**4.** Procédé selon la revendication 1, où l'hydrocarbure aromatique est le benzène.

**5.** Procédé selon la revendication 1, où l'oléfine est un membre sélectionné parmi le groupe constitué en l'éthylène, le propylène, le but-1-ène, le trans-but-2-ène, et le cis-but-2-ène ou leurs mélanges.

**6.** Procédé selon la revendication 1, où le produit de l'alkylation est le cumène ou l'éthylbenzène.

**7.** Procédé pour la transalkylation d'un hydrocarbure aromatique, où un excès stoéchiométrique d'un hydrocarbure aromatique est contacté avec un hydrocarbure polyalkyle-aromatique dans des conditions au moins partiellement en phase liquide avec un catalyseur comprenant une zéolite-bêta qui a été préparée par un procédé comprenant les étapes suivantes :

(a) préparer un mélange réactionnel comprenant au moins une source active de silice, éventuellement au moins une source active d'alumine, un agent structurant organique capable de former ladite zéolite cristalline, et assez d'eau pour former ledit mélange ; et
(b) chauffer ledit mélange réactionnel dans des conditions de récristallisation et en l'absence d'une phase liquide externe pendant un temps suffisant pour former un matériau cristallin contenant des cristaux de ladite zéolite, où lesdits cristaux de zéolite ont un rapport silice/alumine supérieur à 15 ;
où ledit catalyseur a une capacité d'adsorption d'eau supérieure à 12 pour cent en poids, une microporosité supérieure à 13 cm$^3$/g et une acidité totale supérieure à 0,7 mmol/g ;
où l'acidité totale est mesurée par désorption programmée de température d'isopropylamine entre 280° et 500°C, exécutée par la procédure décrite dans l'Exemple 6 dans la description ; et
où la capacité d'adsorption d'eau est mesurée en utilisant le procédé de H Corr-Purcell-Meiboom-Gill.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3308069 A
- US 4891458 A
- US 5081323 A
- US 5558851 A
- US RE28341 E
- EP 159846 A
- EP 159847 A
- US 4508837 A

**Non-patent literature cited in the description**

- **T.C FARRAR ; E.D. BECKER.** Pulse and Fourier Transform NMR. Academic Press, 1971